(19) Europäisches Patentamt
European Patent Office
Office européen des brevets

(11) Veröffentlichungsnummer: **0 025 935**
**B1**

(12) **EUROPÄISCHE PATENTSCHRIFT**

(45) Veröffentlichungstag der Patentschrift: **22.12.82**

(51) Int. Cl.³: **C 07 D 307/32**, C 07 D 307/30

(21) Anmeldenummer: **80105379.4**

(22) Anmeldetag: **09.09.80**

(54) **Verfahren zur Herstellung von 5-(2,2,2-Trihalogenethyl)-dialkyl-tetrahydrofuran-2-onen.**

(30) Priorität: **19.09.79 DE 2937836**

(43) Veröffentlichungstag der Anmeldung:
**01.04.81 Patentblatt 81/13**

(45) Bekanntmachung des Hinweises auf die Patenterteilung:
**22.12.82 Patentblatt 82/51**

(84) Benannte Vertragsstaaten:
**AT BE CH DE FR GB IT LI NL SE**

(56) Entgegenhaltungen:
**DE-A-2 813 336**
**PATENT ABSTRACTS OF JAPAN, unexamined applications, Sektion C, Basel 1, Nr. 131, 28. Oktober 1977, The Patent Office Japanese Government, Seite 2880 C 77. Kokai-nr. 52-83456**
**PATENT ABSTRACTS OF JAPAN, unexamined applications, Sektion C, Band 1, Nr. 131, 28. Oktober 1977, The Patent Office Japanese Government, Seite 2880 C 77. Kokai-Nr. 52-83457**
**PATENT ABSTRACTS OF JAPAN, unexamined applications, Sektion C, Band 1, Nr. 131, 28. Oktober 1977, The Patent Office Japanese Government, Seite 2881 C 77. Kokai-Nr. 52-83458.**
**PATENT ABSTRACTS OF JAPAN, unexamined applications, Sektion C, Band 1, Nr. 131, 28. Oktober 1977, The Patent Office Japanese Government, Seite 2881 C 77. Kokai-Nr. 52-83459**

(73) Patentinhaber: **BASF Aktiengesellschaft, Carl-Bosch-Strasse 38, D-6700 Ludwigshafen (DE)**

(72) Erfinder: **Kropp, Rudolf, Sprottauer Strasse 2, D-6703 Limburgerhof (DE)**
Erfinder: **Fischer, Martin, Dr., Elbingerweg 1, D-6700 Ludwigshafen (DE)**
Erfinder: **Halbritter, Klaus, Dr., Schwarzwaldstrasse 19, D-6800 Mannheim (DE)**

(56) Entgegenhaltungen:
**PATENT ABSTRACTS OF JAPAN, unexamined applications, Sektion C, Band 1, Nr. 131, 28. Oktober 1977, The Patent Office Japanese Government, Seite 2927 C 77. Kokai-Nr. 52-83734.**
**Die Akte enthält technische Angaben, die nach dem Eingang der Anmeldung eingereicht wurden und die nicht in dieser Patentschrift enthalten sind.**

EP 0 025 935 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Verfahren zur Herstellung von 5-(2,2,2-Trihalogenethyl)-dialkyl-tetrahydrofuran-2-onen.

Die Erfindung betrifft ein Verfahren zur Herstellung von 5-(2,2,2-Trihalogenethyl)-4,4-dialkyl-tetrahydrofuran-2-onen durch Umsetzung von N,N-disubstituierten Carbonsäureamiden mit Tetrahalogenmethanen und anschliessende Hydrolyse der so erhaltenen Iminiumsalze.

Die Herstellung von 5-(2,2,2-Trichlorethyl)-4,4-dimethyl-tetrahydrofuran-2-onen durch Addition von 3,3,3-Trichlorpropanol an 3,3-Dimethylacrylsäureester und anschliessende Dehydrochlorierung ist bekannt (JP-OS 77/83456). Von Nachteil bei diesem Verfahren ist neben der geringen Ausbeute die schlechte Zugänglichkeit des Ausgangsproduktes 3,3,3-Trichlorpropanol.

Es wurde nun gefunden, dass man 5-(2,2,2-Trihalogenethyl)-4,4-dialkyl-tetrahydrofuran-2-one der Formel I

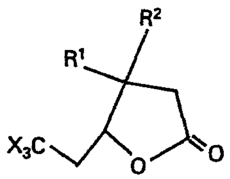

(I)

in der $R^1$ und $R^2$ je einen Alkylrest mit 1 bis 4 Kohlenstoffatomen und X Halogen bedeuten, in vorteilhafter Weise erhält, wenn man ein Carbonsäureamid der Formel II

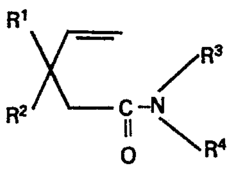

(II)

in der $R^1$ und $R^2$ je einen Alkylrest mit 1 bis 4 Kohlenstoffatomen und $R^3$ und $R^4$ je einen Alkylrest mit 1 bis 4 Kohlenstoffatomen, einen Aralkylrest mit 7 bis 9 Kohlenstoffatomen oder einen Arylrest mit 6 bis 10 Kohlenstoffatomen bedeuten oder zusammen mit dem Stickstoffatom, dessen Substituenten sie sind, einen 5- oder 6-gliedrigen gesättigten Ring bilden, der noch ein weiteres Heteroatom enthalten kann, mit einem Tetrahalogenmethan der Formel III

$CX_4$ (III)

in der X Halogen bedeutet, in Gegenwart eines Initiators und eines organischen Verdünnungs- oder Lösungsmittels, bei einer Temperatur im Bereich zwischen Raumtemperatur und 120 °C zu einem Iminiumsalz der Formel IV

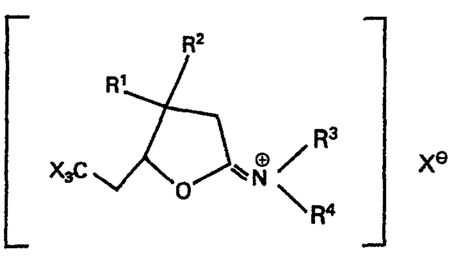

(IV)

in der $R^1$, $R^2$, $R^3$, $R^4$ und X die obengenannten Bedeutungen haben, umsetzt und dieses Iminiumsalz anschliessend hydrolysiert.

Nach dem erfindungsgemässen Verfahren lassen sich 5-(2,2,2-Trihalogenethyl)-4,4-dialkyl-tetrahydrofuran-2-one herstellen, die als Zwischenprodukte für die Synthese insektizider Wirkstoffe dienen. Die Verbindungen lassen sich durch Halogenwasserstoffabspaltung, beispielsweise mit Alkalialkoholat oder durch Erhitzen, und durch Behandeln mit einem anorganischen Säurehalogenid, beispielsweise einem Thionylhalogenid, und anschliessende Umsetzung mit einer Base in 2-(2,2-Dihalogenvinyl)-3,3-dialkyl-cyclopropancarbonsäureester überführen (DE-OS 26 21 831), von denen insbesondere die 2-(2,2-Dihalogenvinyl)-3,3-dimethyl-cyclopropancarbonsäurealkylester Ausgangsprodukte für die Synthese insektizider Wirkstoffe aus der Klasse der Pyrethroide sind. Die Synthese der 5-(2,2,2-Trihalogenethyl)-4,4-dialkyl-tetrahydrofuran-2-one nach dem erfindungsgemässen Verfahren ist besonders vorteilhaft und wirtschaftlich, da die gewünschten Produkte, ausgehend von gut zugänglichen Ausgangsmaterialien, auf technisch einfach durchführbare Weise in guten Ausbeuten erhalten werden.

Die als Ausgangsstoffe verwendeten Carbonsäureamide der Formel II sind bekannt und können in Anlehnung an bekannte Verfahren hergestellt werden (JP-OS 77/83 411; DE-OS 27 32 213). Die Ausgangsstoffe der Formel II können auch durch Umsetzung von Acetamidacetalen oder Ketenacetalaminalen mit 3-Methyl-but-2-en-1-ol erhalten werden.

Bevorzugte Ausgangsstoffe der Formel II sind 3,3-Dimethyl-pent-4-ensäure-N,N-dimethylamid, 3,3-Diäthyl-pent-4-en-säure-N,N-dimethylamid, 3-Methyl-3-n-propyl-pent-4-ensäure-N,N-dimethylamid, 3,3-Dimethyl-pent-4-ensäure-N-methyl-N-phenylamid, 3,3-Dimethyl-pent-4-ensäure-N-methyl-N-benzylamid, 3,3-Dimethyl-pent-4-ensäure-pyrrolidid und 3,3-Dimethyl-pent-4-ensäure-morpholid.

Für die Umsetzung geeigente Tetrahalogenmethane der Formel III sind beispielsweise Tetrachlormethan, Tetrabrommethan und Bromtrichlormethan.

Die Ausgangsstoffe der Formel II und III können in stöchiometrischer Menge umgesetzt werden. Es ist jedoch von Vorteil, den Ausgangsstoff der Formel III im Überschuss einzusetzen, d.h. im allgemeinen in einer Menge von 1 bis 20 Mol, vorteilhaft 3 bis 10 Mol, pro Mol Ausgangsstoff der Formel II.

Als Initiatoren für die Umsetzung der Carbonsäureamide mit den Tetrahalogenmethanen zu Iminiumsalzen eignen sich organische Peroxide oder Perester, wie Di-tert.-butylperoxid, Dibenzoylperoxid oder 2-Ethyl-hexansäure-tert.-butylperester, Azo-bis-isobuttersäurenitril, Redoxsysteme, die Eisen- und/oder Kupferionen, wie $Fe^{3+}$/

$Fe^{2+}$, $Cu^{2+}/Cu^{1+}$, $Cu^{2+}/Fe^{2+}$, enthalten sowie UV-Licht. Pro Mol Carbonsäureamid der Formel II werden 0,01 bis 0,5 Mol, vorzugsweise 0,05 bis 0,2 Mol, Initiator zugesetzt.

Als organische Verdünnungs- oder Lösungsmittel kommen gegebenenfalls chlorierte aliphatische und aromatische Kohlenwasserstoffe, wie Heptan, Cyclohexan, Benzol, Toluol oder Chlorbenzol, in Betracht. Zweckmässigerweise verwendet man jedoch einen Überschuss an Tetrahalogenmethan der Formel III als Lösungsmittel. Die Menge an Lösungsmittel pro Verbindung der Formel II kann in weiten Bereichen variiert werden; sie kann 100 bis 2000, zweckmässigerweise 300 bis 1000 Gew.-%, bezogen auf Carbonsäureamid, betragen.

Die Umsetzung der Carbonsäureamide der Formel II mit Tetrahalogenmethanen der Formel III zu Iminiumsalzen der Formel IV wird bei einer Temperatur im Bereich zwischen Raumtemperatur und 120 °C drucklos oder unter Druck, diskontinuierlich oder kontinuierlich, durchgeführt.

Setzt man mit Tetrachlormethan um, so verläuft die Reaktion bei einer Temperatur im Bereich zwischen 90 und 120 °C, vorzugsweise zwischen 100 und 110 °C, verwendet man Tetrabrommethan oder Bromtrichlormetham, so arbeitet man bei einer Temperatur zwischen Raumtemperatur und 120 °C, vorzugsweise zwischen 20 und 100 °C.

Die durch Umsetzung der Carbonsäureamide der Formel II mit Tetrahalogenmethanen der Formel III erhaltenen Iminiumsalze der Formel IV sind neu. In dieser Formel bedeutet X Halogen, vorzugsweise Chlor oder Brom, wobei sowohl die Halogensubstituenten der Ethylgruppe als auch das Anion verschieden sein können. $R^1$ und $R^2$ in Formel IV bedeuten je einen Alkylrest mit 1 bis 4 Kohlenstoffatomen, insbesondere Methyl. $R^3$ und $R^4$ können für einen Alkylrest mit 1 bis 4 Kohlenstoffatomen, insbesondere Methyl, Äthyl, Isopropyl, einen Aralkylrest mit 7 bis 9 Kohlenstoffatomen, insbesondere Benzyl, oder einen Arylrest mit 6 bis 10 Kohlenstoffatomen, insbesondere Phenyl, stehen. $R^3$ und $R^4$ können auch zusammen mit dem Stickstoffatom, dessen Substituenten sie sind, einen 5- oder 6-gliedrigen Ring bilden, der noch ein weiteres Heteroatom, insbesondere Sauerstoff, enthalten kann. Beispiele für solche Ringe sind Pyrrolidin, Piperidin und Morpholin.

Die Iminiumsalze der Formel IV lassen sich durch Behandeln mit der 1- bis 20-fachen, vorzugsweise 5- bis 10-fachen, Gewichtsmenge Wasser bei einer Temperatur im Bereich zwischen 20 und 150 °C, vorzugsweise zwischen 80 und 100 °C, in die Verbindungen der Formel I überführen, wobei die Umsetzung drucklos oder unter Druck erfolgen kann. Diese Hydrolyse kann in Gegenwart des organischen Verdünnungs- oder Lösungsmittels durchgeführt werden, in dessen Anwesenheit die Synthese des Iminiumsalzes durchgeführt wurde. Die dann anfallenden Tetrahydrofuranone der Formel I können in üblicher Weise durch Abtrennung von der wässrigen Phase durch Extraktion aus dem wässrigen Reaktionsgemisch isoliert und durch Destillation oder Kristallisation gereinigt werden.

Das erfindungsgemässe Verfahren wird durch die folgenden Beispiele erläutert.

Beispiel 1

Eine Mischung aus 250 ml Cylohexan, 15,5 g (0,1 Mol) 3,3-Dimethyl-pent-4-ensäure-N,N-dimethylamid, 33,2 g (0,1 Mol) Tetrabrommethan und 2,4 g (0,01 Mol) Dibenzoylperoxid wird 5 Stunden lang bei 80 bis 85 °C gerührt. Nach dem Abdestillieren des Cyclohexans und 5 g nicht umgesetztem 3,3-Dimethyl-pent-4-ensäure-N,N-dimethylamids erhält man als kristallinen Rückstand N,N-Dimethyl-[5-(2,2,2-tribromethyl)-4,4-dimethyl-tetrahydrofuryl-2]-iminiumbromid. Der Rückstand wird in 100 ml Wasser 1 Stunde lang bei 80 bis 90 °C gerührt und mit Methylenchlorid extrahiert. Nach dem Abdestillieren des Methylenchlorids erhält man 19 g 5-(2,2,2-Tribromethyl)-4,4-dimethyl-tetrahydrofuran-2-on vom Schmp. 102 bis 102,5 °C.

$C_8H_{11}O_2Br_3$ (379)
Ber.: C 25,33%; H 2,9%; Br 63,3%
Gef.: C 25,5 %; H 2,9%; Br 63,2%
NMR (80 MHz): 1,05 ppm (s; 3H)
1,25 ppm (s; 3H)
2,4 ppm (d; 2H)
3,32 ppm (d; 2H)
4,3 ppm (t; 1H)

Beispiel 2

Eine Lösung von 31 g (0,2 Mol) 3,3-Dimethyl-pent-4-ensäure-N,N-dimethylamid in 120 g (0,6 Mol) Bromtrichlormethan und 200 ml Tetrachlormethan wird 15 Stunden bei 20 °C mit einer 300 W-Quecksilber-Hochdrucklampe bestrahlt. Nach dem Abdestillieren der flüchtigen Anteile unter vermindertem Druck bei maximal 30 °C wird das zurückbleibende Reaktionsgemisch mit 300 ml Petroläther gerührt. Dabei werden 51 g N,N-Dimethyl-[5-(2,2,2-trichlorethyl)-4,4-dimethyl-tetrahydrofuryl-2]-iminiumbromid ausgefällt. Die hygroskopische Substanz schmilzt bei 190 bis 191 °C.

$C_{10}H_{17}ONCl_3Br$ (354)
Ber.: N 3,96%; Cl 30,09%; Br 22,6% (ionogen)
Gef.: N 4,0 %; Cl 29,2%; Br 23,8% (ionogen)
NMR (80 MHz): 1,25 ppm (s; 3H)
1,4 ppm (s; 3H)
2,9–4,0 ppm (mp; 4H)
3,45 ppm (s; 3H)
3,6 ppm (s; 3H)
5,3 ppm (d; 1H)

Beispiel 3

Zu einer Lösung von 120 g (0,6 Mol) Bromtrichlormethan in 250 ml Cyclohexan gibt man unter Rühren bei 85 °C kontinuierlich 31 g (0,2 Mol) 3,3-Dimethyl-pent-4-ensäure-N,N-dimethylamid und 4,6 g (0,02 Mol) 2-Ethyl-hexansäure-tert.-butylperester. Man rührt noch 2 Stunden bei 85 °C nach. Es kristallisieren 28 g N,N-Dimethyl-[5-(2,2,2-trichlorethyl)-4,4-dimethyl-tetrahydrofuryl-2]-iminiumbromid vom Schmp. 190 bis 191 °C aus.

Nach dem Einengen des Filtrats und Zugabe von 200 ml Petroläther gewinnt man weitere 31 g N,N-Dimethyl-[5-(2,2,2-trichlorethyl)-4,4-dimethyltetrahydrofuryl-2]-iminiumbromid.

Beispiel 4

Eine Lösung von 31 g (0,2 Mol) 3,3-Dimethylpent-4-ensäure-N,N-dimethylamid und 66,4 g (0,2 Mol) Tetrabrommethan in 260 ml Tetrachlormethan wird 7 Stunden lang bei 20 °C mit einer 300 W-Quecksilber-Hochdrucklampe bestrahlt. Nach dem Abdestillieren der flüchtigen Anteile aus dem Reaktionsgemisch erhält man einen kristallinen Rückstand. Durch Aufrühren mit 100 ml Chloroform, Absaugen und Trocknen gewinnt man 30 g (0,06 Mol) N,N-Dimethyl-[5-(2,2,2-tribromethyl)-4,4-dimethyl-tetrahydrofuryl-2]-iminiumbromid vom Schmp. 169 bis 169,5 °C.

$C_{10}H_{17}ONBr_4$ (487)
Ber. C 24,67%; H 3,52%, N 2,88%; Br (gesamt) 65,65%; Br (ionogen ) 16,4%
Gef.: C 24,8%; H 3,6%; N 2,9%; Br (gesamt) 65,1%; Br (ionogen) 16,1%.
NMR (80 MHz): 1,1 ppm (s; 3H)
1,28 ppm (s; 3H)
3,2 ppm (d; 2H)
3,24 ppm (s; 3H)
3,26 ppm (s; 3H)
3,55 ppm (d; 2H)
4,92 ppm (t; 1H)

Beispiel 5

26 g N,N-Dimethyl-[5-(2,2,2-trichlorethyl)-4,4-dimethyl-tetrahydrofuryl-2]-iminiumbromid wird 3 Stunden lang auf 130 °C bei einem Druck von 25 mbar erhitzt. Man erhält 21 g N,N-Dimethyl-[5-(2,2-dichlorvinyl)-4,4-dimethyl-tetrahydrofuryl-2]-iminiumbromid, das durch Erhitzen mit 80 ml Wasser auf 90 °C nach 2 Stunden 13 g 5-(2,2-Dichlorvinyl)-4,4-dimethyl-tetrahydrofuran-2-on ergibt.

Beispiel 6

In einem Rührautoklav aus rostfreiem Stahl werden 77 g (0,5 Mol) 3,3-Dimethyl-pent-4-ensäure-N,N-dimethylamid, 130 g (0,84 Mol) Tetrachlormethan, 35 g Acetonitril, 0,85 g (0,005 Mol) $CuCl_2 \cdot 2H_2O$ und 1 g Diäthylammoniumchlorid 12 Stunden lang bei 100 °C und unter einem Druck von 5 bar gerührt. Aus dem Reaktionsgemisch werden bei vermindertem Druck Acetonitril und überschüssiges Tetrachlormethan abdestilliert. Zurück bleiben 163 g einer halbkristallinen Masse, die in Tetrachlormethan unlöslich ist und nach Elementaranalyse und Massenspektrum im wesentlichen aus N,N-Dimethyl-[5-(2,2,2-trichlorethyl)-4,4-dimethyl-tetrahydrofuryl-2]-iminiumchlorid besteht. Das Produkt wird bei 20 °C mit 500 ml Wasser behandelt und mit Tetrachlormethan extrahiert. Nach dem Abziehen des Lösungsmittels erhält man durch Destillation 24 g 5-(2,2,2-Trichlorethyl)-4,4-dimethyl-tetrahydrofuran-2-on vom Sdp. 111 bis 115 °C/0,013 mbar und Schmp. 56 bis 58 °C.

$C_8H_{11}O_2Cl_3$ (246)
Ber.: C 39,13%; H 4,52%; O 13,03%; Cl 43,32%
Gef.: C 39,6 %; H 4,8 %; O 13,1 %; Cl 42,6 %
NMR (100 MHz): 1,02 ppm (s; 3H)
1,21 ppm (s; 3H)
2,38 ppm (dd; 2H)
2,98 ppm (d; 2H)
4,45 ppm (t; 1H)

**Patentanspruch**

Verfahren zur Herstellung von 5-(2,2,2-Trihalogenethyl)-4,4-dialkyl-tetrahydro-furan-2-onen der Formel I

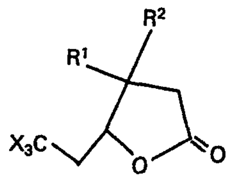

(I)

in der

$R^1$ und $R^2$ je einen Alkylrest mit 1 bis 4 Kohlenstoffatomen und

X Halogen bedeuten, dadurch gekennzeichnet, dass man ein Carbonsäureamid der Formel II

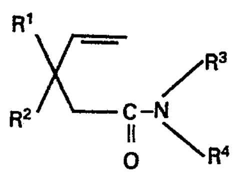

(II)

in der

$R^1$ und $R^2$ je einen Alkylrest mit 1 bis 4 Kohlenstoffatomen und

$R^3$ und $R^4$ je einen Alkylrest mit 1 bis 4 Kohlenstoffatomen, einen Aralkylrest mit 7 bis 9 Kohlenstoffatomen oder einen Arylrest mit 6 bis 10 Kohlenstoffatomen bedeuten oder zusammen mit dem Stickstoffatom, dessen Substituenten sie sind, einen 5- der 6-gliedrigen gesättigten Ring bilden, der noch ein zweites Heteroatom enthalten kann, mit einem Tetrahalogenmethan der Formel II

$CX_4$ (III)

in der X Halogen bedeutet, in Gegenwart eines Initiators und eines organischen Verdünnungs- oder Lösungsmittels bei einer Temperatur im Bereich zwischen Raumtemperatur und 120 °C zu einem Iminiumsalz der Formel IV

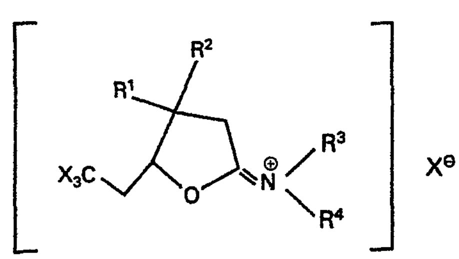

(IV)

in der
$R^1$, $R^2$, $R^3$, $R^4$ und X die genannten Bedeutungen haben, umsetzt und dieses Iminiumsalz anschliessend hydrolysiert.

**Revendication**

Procédé de préparation de 5-(2,2,2-trihalogèno-éthyl)-4,4-dialkyl-tétrahydro-furan-2-ones de formule I

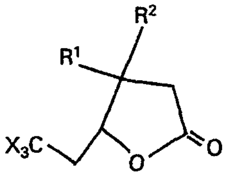

(I)

dans laquelle
$R^1$ et $R^2$ représentent chacun un reste alkyle ayant 1 à 4 atomes de carbone, et
X un halogène, caractérisé par le fait qu'en présence d'un initiateur et d'un diluant ou solvant organique, à une température comprise entre la température ambiante et 120 °C, on fait réagir un amide d'acide carboxylique de formule II

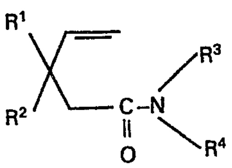

(II)

dans laquelle
$R^1$ et $R^2$ représentent chacun un reste alkyle ayant 1 à 4 atomes de carbone, et
$R^3$ et $R^4$ représentent chacun un reste alkyle ayant 1 à 4 atomes de carbone, un reste aralkyle ayant 7 à 9 atomes de carbone ou un reste aryle ayant 6 à 10 atomes de carbone, ou forment, avec l'atome d'azote dont ils sont des substituants, un noyau saturé à 5 ou 6 chaînons, qui peut encore contenir un second hétéroatome, avec un tétrahalogénométhane de formule III

$CX_4$ (III)

dans laquelle X représente un halogène, pour obtenir un sel d'iminium de formule IV

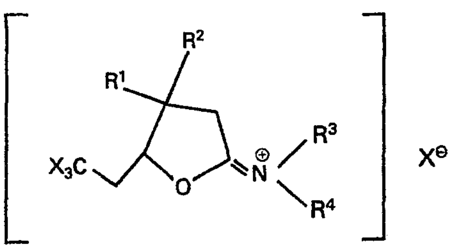

(IV)

dans laquelle
$R^1$, $R^2$, $R^3$, $R^4$ et X ont les significations susmentionnées, et on hydrolyse ensuite ce sel d'iminium.

**Claim**

A process for the preparation of a 5-(2,2,2-trihaloethyl)-4,4-dialkyl-tetrahydro-furan-2-one of the formula I

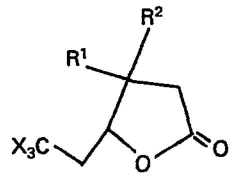

(I)

where $R^1$ and $R^2$ are each alkyl of 1 to 4 carbon atoms and X is halogen, wherein a carboxylic acid amide of the formula II

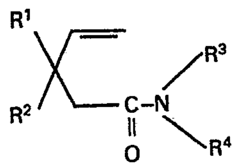

(II)

where $R^1$ and $R^2$ are each alkyl of 1 to 4 carbon atoms and $R^3$ and $R^4$ are each alkyl of 1 to 4 carbon atoms, aralkyl of 7 to 9 carbon atoms or aryl of 6 to 10 carbon atoms, or together with the nitrogen atom on which they are present as substituents form a 5-membered or 6-membered saturated ring which may contain a second hetero-atom, is reacted with a carbon tetrahalide of the formula III

$CX_4$ (III)

where X is halogen, in the presence of an initiator and of an organic diluent or solvent at from room temperature to 120 °C, to give an iminium salt of the formula IV

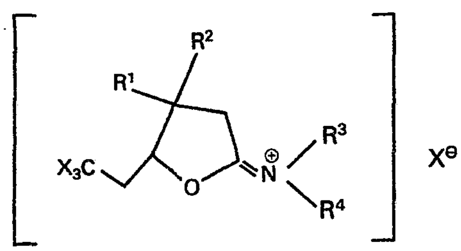

(IV)

where $R^1$, $R^2$, $R^3$, $R^4$ and X have the above meanings, and this iminium salt is subsequently hydrolyzed.